**Europäisches Patentamt**

**European Patent Office**

**Office européen des brevets**

(19)

(11) Veröffentlichungsnummer : **0 278 954 B1**

(12) # EUROPÄISCHE PATENTSCHRIFT

(45) Veröffentlichungstag der Patentschrift :
**22.01.92 Patentblatt 92/04**

(51) Int. Cl.$^5$ : **A61M 5/14**

(21) Anmeldenummer : **87903853.7**

(22) Anmeldetag : **13.06.87**

(86) Internationale Anmeldenummer :
**PCT/DE87/00271**

(87) Internationale Veröffentlichungsnummer :
**WO 88/00065 14.01.88 Gazette 88/02**

(54) **GASBETRIEBENE DOSIERVORRICHTUNG.**

(30) Priorität : **30.06.86 DE 3621846**

(43) Veröffentlichungstag der Anmeldung :
**24.08.88 Patentblatt 88/34**

(45) Bekanntmachung des Hinweises auf die
Patenterteilung :
**22.01.92 Patentblatt 92/04**

(84) Benannte Vertragsstaaten :
**CH FR GB IT LI SE**

(56) Entgegenhaltungen :
**EP-A- 0 209 644
BE-B- 1 099 132
DE-A- 2 239 432
GB-A- 2 054 381**

(56) Entgegenhaltungen :
**US-A- 4 267 836
MEDICAL PROGRESS through Technology,
Band 6, No 4, 1979, Springer-Verlag 1979, (Berlin, DE), P.J. Blachshear et al.:" The implantable infusion pump: A new concept in drug delivery",see pages 149-161**

(73) Patentinhaber : **WINSEL, August, Prof. Dr.
Fasanenstrasse 8a
W-6233 Kelkheim (DE)**

(72) Erfinder : **WINSEL, August, Prof. Dr.
Fasanenstrasse 8a
W-6233 Kelkheim (DE)**

(74) Vertreter : **Fischer, Franz Josef
BOVARD AG Patentanwälte VSP
Optingenstrasse 16
CH-3000 Bern 25 (CH)**

EP 0 278 954 B1

**Beschreibung**

Die vorliegende Erfindung betrifft ein Vorrichtung zur Injektion oder Infusion einer pharmazeutischen Lösung, mit einem Speicher, der die pharmazeutische Lösung beinhaltet und einer gasentwickelnden Zelle, bei der das durch einen elektrischen Strom erzeugte Wasserstoff- bzw. Sauerstoffgas die Lösung aus dem Speicher mit Hilfe eines beweglichen Kolbens oder einer flexiblen Membran verdrängt.

Eine derartige Vorrichtung ist aus der DE-A-2 239 432 bekannt, die ein implantierbare Gerät für die Medikamentzufuhr in den menschlichen oder tierischen Körper vorschlägt, das sich in einer Ausführungsform der Volumenänderung durch elektrolytische Gaserzeugung zum Vortrieb eines Kolbens oder einer Membran bedient. Dabei ist vorgesehen, die dazu notwendige Energie aus einer Primärzelle bereitzustellen.

In der DP-Anmeldung P 35 32 335.3 ist eine galvanische Zelle beschrieben, in der in einer zum Strom äquivalenten Menge Wasserstoff bzw. Sauerstoff erzeugt werden. Insbesondere kann die entwicklung von Wasserstoff ohne Zuführung von äußerer Energie erfolgen, wenn man eine Zink-elektrode in Kombination mit einer Wasserstoff-Elektrode verwendet, die beide in einem vorzugsweise alkalischen Elektrolyten betrieben werden. Durch Kurzschluß der Zelle über einen konstanten oder auch veränderlichen Widerstand lassen sich beliebige zeitliche Wasserstofferzeugungsprofile realisieren. Da dieses Geschehen auf kleinstem Raume erfolgt, ist die Methode besonders zur Injektion von pharmazeutischen Lösungen in den Körper hinein geeignet. Da die erzeugte Wasserstoffmenge äquivalent zum elektrischen Strom ist, können wegen dessen außerordentlich empfindlicher Dosierbarkeit kleinste Flüssigkeitsmengen mit entsprechender Genauigkeit gefördert werden.

In der Human-Medizin wie auch in der Tier-Medizin spielt die therapeutische Behandlung durch Injektion oder Infusion von Arzneimitteln in die Körperflüssigkeit oder in das Gewebe eine bedeutende Rolle. Injektionen werden vom Arzt oder von besonders geschultem Personal vorgenommen und können daher besonders bei akuter, nichtstationärer Behandlung nur von Zeit zu Zeit vorgenommen werden. Die Injektion appliziert die wirksamen Substanzen stoßweise; der Körper muß sie also zur Erzielung einer Langzeitwirkung zwischenspeichern. Die Injektion darf nicht überdimensionieren und muß doch eine gewisse Zeit die Wirksamkeit der injizierten Substanz im Körper garantieren. Das erfordert sowohl Kompromisse bei dem chemischen Aufbau der Wirksubstanzen wegen deren Resorbierbarkeit vom Körper, der Zusammensetzung der Injektionslösung und deren therapeutischen Anwendung nach Häufigkeit und Menge der Injektion. Wünschenswert jedoch ist eine den jeweiligen Bedürfnissen angepaßte ständig ablaufende Injektion der Wirkstoffe in Form einer gesteuerten, wenn nicht gar geregelten Injektion.

Bekanntestes Beispiel einer derartigen kontinuierlichen Injektionstherapie ist die Behandlung von Diabetikern mit Insulinlösung. Durch kontinuierliche Injektion lassen sich mit wesentlich geringeren Insulinmengen bessere Behandlungs erfolge erzielen als durch die bisherige schubweise Zuführung im Rhythmus der Mahlzeiten. Deshalb hat man Mikropumpen entwickelt, die mit Hilfe einer Batterie als Energievorrat ständig eint kleine Insulinmenge evt. programmgesteuert nach dem jeweiligen zeitlichen Bedarf in den Körper dosieren. Das Endziel dieser Entwicklung ist, die Glukosekonzentration im Blut mit geeigneten Sensoren ständig zu messen und die zugeführte Insulinmenge damit zu regeln, so daß der Körper stets die Insulinmenge seinem Bedarf entsprechend zugeführt bekommt. Allerdings sind verläßlich arbeitende langzeitstabile Glucosefühler noch nicht verfügbar.

Es wurde nun gefunden, daß sich gasentwickelnde Zellen besonders gut zur Förderung von therapeutisch wirksamen flüssigkeiten in den menschlichen oder tierischen Körper eignen.

Ein Problem entsteht lediglich dadurch, daß das Gasvolumen im Gegensatz zum Volumen der geförderten Flüssigkeit temperatur- und druckabhängig ist. Dieses hat zur Folge, daß z. B. ein Diabetes-Kranker mit einer auf Normaldruck eingestellten, kontinuierlichen oder zeitgesteuerten Insulinversorgung beim Betreten eines Flugzeugs eint Korrektur an der Dosiervorrichtung vornehmen muß, mit der der auf eine geometrische Höhe von 3 000 m eingestellte Kabinendruck berücksichtigt wird. eine solche Korrektur ist ohne weiteres möglich, wenn man mit Hilfe eines einfachen Rechnerchips aufgrund der Zustandsgleichung der Gase das erzeugte Gasvolumen auf den wirklichen Druck und die herrschende Temperatur umrechnet. Dieses aber erhöht den Aufwand im elektronischen Bereich des Gerätes und macht den einsatz einer, wenn auch kleinen, zusätzlichen Spannungsquelle in Form einer Batterie notwendig. Aufgabe der Vorliegenden Erfindung ist es, eine Vorrichtung der genannten Art so auszubilden, daß die Dosierbarkeit genauer und Kontrollierbarer ist und weitgehend unabhängig von den durch Temperatur und Luftdruck gegebenen Umweltbedingungen.

Es wurde nun gefunden, daß man sich vom Umgebungsdruck und von der Umgebungstemperatur weitgehend unabhängig machen kann, wenn man die Oberflächenspannung $\sigma$ der zu fördernden Flüssigkeit als eine konstante Gegendruckquelle und gewissermaßen als Druckstandard benutzt. Bekanntlich hat die Oberflächenspannung dieselbe Einheit in $dyn/cm^2$ wie der Druck. Man kann sie also in der beabsichtigten Funktion dann benutzen, wenn die Flüssigkeit gegen einen durch die Oberflächenspannung bestimmten Gegendruck gefördert wird. Dieses geschieht durch Auflösung des geförderten Flüssigkeitsstroms in einen Strom diskreter Flüssigkeitstropfen, der ggf. zu einem kontinuierlichen Strom wiedervereinigt werden kann.

Gegendruck der Förderung in die Tropfenstrecke hinein ist der Laplace-Druck$_{PL}$ der Tröpfchen vom Radius R, gegeben durch die Gleichung

$$PL = 2\,\sigma\,/R.$$

In dieser Gleichung ist $\sigma$ die Oberflächenspannung der zu fördernden Flüssigkeit, gemessen gegen eine Atmosphäse des gleichen dampfförmigen Mediums mit eingestelltem Partialdruck-Gleichgewicht. Besteht die zu fördernde Flüssigkeit nicht aus einer einheitlichen Lösung, sondern aus einer Lösung mehrerer Komponenten, so ist deren Dampfdruck im wesentlichen durch die am niedrigsten siedende Komponente bestimmt. Das gleiche gilt für die Förderung von Emulsionen fester und flüssiger Stoffe in Emulgationsflüssigkeiten, ggf. unter Zuhilfenahme von Emulgatoren. Auch die Förderung von Pasten kann so bewerkstelligt werden, wenn es gelingt, aufgrund deren rheologischer Eigenschaften eine Tropfenstrecke zu erzeugen.

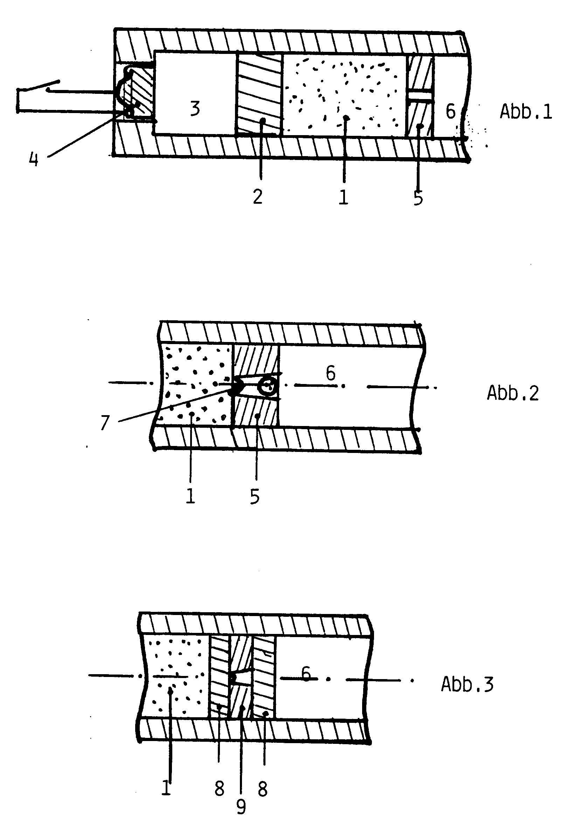

In Abb. 1 ist dargestellt, wie eine Tropfenstrecke erzeugt werden kann. 1 ist der Vorratsbehälter für die zu fördernde Flüssigkeit, 2 ist der Kolben oder eine als Kolben wirkende Membran, die von dem sich entwickelnden Gas 3 der Gasentwicklungszelle 4 einen allseitigen Druck auf die zu fördernde Flüssigkeit in 1 ausübt. 6 ist der Raum unter Umgebungsdruck, in den gefördert wird.

Die Ausbildung der Tropfenstrecke erfolgt dadurch, daß man die zu fördernde Flüssigkeit in der Ausströmungsleitung durch eine lyophobe Lochblende 5 oder eine lyophobe Düse mit in etwa kegelförmigem Öffnungsquerschnitt laufen läßt, wobei die Kegelspitze auf den Förderbehälter 1 hinweist. Dieses ist in Abb. 2 dargestellt. Unter dem Einfluß des Überdrucks in 1 wird die Flüssigkeit unter Ausbildung einer kugelkalottenförmigen Oberfläche in die Düse hineingedrückt, bis sich nach Erreichen der Halbkugelform ein kugelförmiger Tropfen absondert und in den Raum 6 hineinläuft. Befindet sich im Raum 6 eine saugfähige poröse Substanz, so wird der Tropfen wie von einem Schwamm aufgenommen und weiter transportiert.

Eine ähnliche Wirkung hat die Anordnung in Abb. 3, die aus einer lyophoben Lochblende 9 zwischen zwei lyophilen porösen Körpern 8 besteht. Auch diese Anordnung stellt eine Druckstufe dar, die beim Übergang der Flüssigkeit zwischen dem Räumen 1 und 6 überwunden werden muß. Bei der Betrachtung der Abb. 3 erkennt man, daß eine Flüssigkeitsbrücke, die sich unter dem Einfluß des Überdrucks zwischen Raum 1 in der Lochblende ausgebildet hat und die Räume 1 und 6 miteinander verbindet, bei Abfall des Überdrucks den Raum 1 wieder abreißen sollte. Das geht besonders leicht, wenn die Bohrung in der Lochblende mit Gas versorgt werden kann. es hat sich aus diesem Grunde als zweckmäßig erwiesen, die Lochblende aus feinporigem lyophoben Material herzustellen, dann kann dieses feinporige System als "Druckgasspeicher" dienen und das aus dem Lochbereich verdrängte Gas aufnehmen.

Polyethylen, Polypropylen und Polytetrafluorethylen sind Kunststoffe, die sich gegen manche Flüssigkeiten abstoßend, d.h. lyophob verhalten. Man kann aus solchen Kunststoffen feinporige Scheiben herstellen, die selbst bei hohen flüssigkeitsdrucken bis zu 10 bar und mehr nicht überflutet werden. Man kann Lochscheiben der gewünschten Art dadurch herstellen, daß man mechanisch ein feines Loch einbohrt oder mit einem Laserstrahl einen dünnen Kanal einbrennt.

Denkt man sich die an Raum 6 angrenzende lyophile Scheibe 8 zu einem Docht erweitert, der mit dem zu versorgenden Körpergewebe im Kapillardruckgleichgewicht steht, so führt jeder Tropfen, der die durch Lochblende 9 gebildete Druckschleuse überwindet, zum Abgang eines entsprechend großen Volumens der geförderten Flüssigkeit am anderen Ende des Dochtes. Handelt es sich um eine elektrisch leitende Flüssigkeit, so bewirkt die Überwindung der Druckschleuse jedes Mal die Herstellung einer elektrisch leitenden Verbindung zwischen den Räumen 1 und 6, die man zur selbsttätigen Kontrolle des Fördervorganges ausnutzen kann. Eine solche Kontrolle ist jedoch auch mit optischen Methoden möglich, indem man die veränderte Lichtstreuung beim Durchwandern des Tropfens durch die Druckschleuse mißt und registriert. Die lichtleiter- und Optokopplertechnik bietet hierzu mannigfache Möglichkeiten, die nach den vertretbaren Kosten mehr oder weniger komfortabel ausgeführt werden können.

Aus der bisherigen Beschreibung kann man folgern, daß die erfindungsgemäße Druckstufe nicht nur aus einer einzelnen Pore in einer lyophoben Scheibe bestehen kann, sondern daß es sich hierbei um die Gesamtheit aller Poren in einem der zu fördernden Flüssigkeit gegenüber lyophoben Diaphragma handeln kann. Auch in diesem Fall ist der zum Durchdringen des Diaphragmas erforderliche Förderdruck durch die Kapillardepression der Flüssigkeit in diesem Diaphragma bestimmt. Nach dem Durchdringen des Diaphragmas wird die in Oberflächenenergie umgewandelte Druckenergie bei der Bildung größerer zusammenhängender Tropfen oder bei der Erfüllung der Poren einer benetzbaren Scheibe unter Wärmeentwicklung vernichtet.

**Patentansprüche**

1. Vorrichtung zur Injektion oder Infusion einer pharmazeutischen Lösung, mit einem Speicher, der die pharmazeutische Lösung (1) beinhaltet und einer gasentwickelnden Zelle (4), bei der das durch einen elektrischen Strom erzeugte Wasserstoff- bzw. Sauerstoffgas die Lösung (1) aus dem Speicher mit Hilfe eines beweglichen Kolbens oder einer flexiblen Membran (2) verdrängt, **dadurch gekennzeichnet**, daß die Vorrichtung einen eine Pore oder eine Vielzahl von Poren aufweisenden, gegenüber der zu fördernde Lösung lyophoben Körper (5) besitzt, der in der Vorrichtung derart angebracht ist, daß bei Betätigung der Zelle (4), die zu fördernde Lösung durch die Poren des Körpers (5) transportiert wird.

2. Vorrichtung nach Anspruch 1, dadurch gekennzeichnet, daß die Pore eine lyophobe Kapillare ist, die den Förderstrom der Flüssigkeit in einen Strom diskreter Flüssigkeitstropfen aufteilt.

3. Vorrichtung nach Anspruch 1 oder 2, dadurch gekennzeichnet, daß der lyophobe körper (5) auf der Abströmseite mit einer lyophilen Scheibe (8) kommuniziert, die den in diskrete Tropfen verteilten Flüssigkeitsstrom wieder vereinigt.

4. Vorrichtung nach einem der Anspruch 1 bis 3, dadurch gekennzeichnet, daß der lyophobe Körper (5) auf der Zu -und Abströmseite zwischen lyophilen porösen Körpern (8) angeo dnet ist und mit diesen kommuniziert.

## Claims

1. Device for injecting or infusing a pharmaceutical solution, with a reservoir which contains the pharmaceutical solution (1) and a gas developing cell (4), in which the hydrogen or oxygen created by an electric current forces solution (1) out of the reservoir with the aid of a movable piston or a flexible membrane (2), characterized in that the device has an element (5) which is lyophobic with regard to the solution to be displaced, having one pore or a plurality of pores, which is diposed in the device in such a way that when cell (4) is activated, the solution to be displaced is transported through the pores of element (5).

2. The device of claim 1, characterized in that the pore is a lyophobic capillary which divides the flow of liquid displaced into a flow of individual drops of liquid.

3. The device of claim 1 or 2, characterized in that lyophobic element (5) communicates on the downstream side with a lyophilic disk (8) which once again unifies the stream of liquid divided into individual drops.

4. The device of one of claims 1 to 2, characterized in that lyophobic element (5) is disposed on the inflow and outflow side between lyophilic porous elements (8) and communicates with these.

## Revendications

1. Dispositif pour l'injection ou l'infusion d'une solution pharmaceutique, avec un récipient contenant la solution pharmaceutique (1) et une cellule (4) dégageant du gaz, produisant, par l'action d'un courant électrique, de l'hydrogène, respectivement de l'oxygène qui chasse la solution (1) hors du récipient à l'aide d'un piston mobile ou d'une membrane (2), caractérisé en ce que le dispositif comprend un corps lyophobe (5) comportant une pore ou une pluralité de pores et disposé en face de la solution à refouler et fixé dans le dispositif de telle manière que sous l'action de la cellule (4), la solution à refouler est transportée à travers les pores du corps (5).

2. Dispositif selon la revendication 1, caractérisé en ce que la pore est un capillaire lyophobe qui partage le courant de liquide chassé en un courant de gouttes discrètes.

3. Dispositif selon l'une des revendications 1 ou 2, caractérisé en ce que le corps lyophobe (5) communique sur son côté aval avec un disque lyophile qui réunit à nouveau les courants liquides séparés en gouttes discrètes.

4. Dispositif selon l'une des revendications 1 à 3, caractérisé en ce que le corps lyophobe (5) est disposé sur ses côtés amont et aval entre des corps poreux lyophiles (8) et communique avec eux.

Abb.1

Abb.2

Abb.3